# EUROPEAN PATENT APPLICATION

(11) **EP 2 806 000 A1**
(43) Date of publication of application: **26.11.2014**
(21) Application number: 13177236.0
(22) Date of filing: 19.07.2013
(51) Int. Cl.: C09C 1/36, C09C 3/10, C09C 1/04

(54) **Mass preparation method for spherical natural color wax beads**

(30) Priority: 24.05.2013 KR 20130058978; 26.06.2013 KR 20130073388
(71) Applicant: Sunjin Chemical Co., Ltd., Ansan-Si, Gyeonggi-Do 425-090 (KR)
(72) Inventor: Lee, Sung Ho, Seoul 158-070 (KR); Kim, Sang Uk, Incheon 405-260 (KR); Lim, Chang Wan, Gyeonggi-do 426-170 (KR); Jeong, Yeo Jin, Gyeonggi-do 426-894 (KR)
(74) Representative: Ter Meer Steinmeister & Partner

(57) **Abstract**

This invention relates to a mass preparation method of spherical natural color wax beads and according to the invention, the beads have a higher specific gravity than water so that the beads do not flow into the sea and they are biodegradable to fundamentally remarkably reduce marine ecosystem contamination, and it enables mass preparation by significantly improving the previous preparation of spherical natural color wax beads which had difficulties in their mass preparation while using expensive equipment.

## Description

### BACKGROUND OF THE INVENTION

This invention relates to mass preparation method for spherical natural color wax beads, more particularly to a method capable of mass preparationby significantly improving the previous preparation of spherical natural color wax beads which had difficulties in their mass preparation while using expensive equipment.

Spherical beads having a diameter of 10 to 5000 µm have been used in several areas for various purposes in personal products or industrial fields, and in order to meet needs, mass-produced plastic spherical beads through synthesis are widely used.

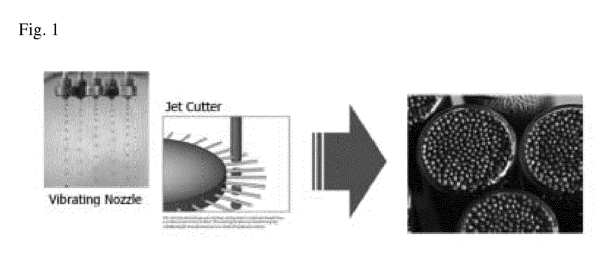

However, these plastic beads might damage human health and as theirspecific gravity is lower than water and it is non-biodegradable, abandoned plastic beads flow into the sea and disturb marine ecosystem, becoming the cause substances of environmental contamination. Recently, due to the problems of the plastic beads, there have been movements to refrain from using plastic beads and to use various natural beads. Of them, there was a method for preparation of spherical beads using a natural substance-derived wax and for example, a method for preparation of spherical wax beads using expensive vibrating nozzle and jet cutter equipment as in Fig. 1 was attempted but this method is not proper for mass preparationand up to now, spherical natural color wax beads are thus being sold at a very expensive price. Therefore, there is an urgent need of mass preparation method of spherical natural color wax beads.

### SUMMARY OF THE INVENTION

Therefore, it is an object of the present invention to provide a method capable of mass preparation of spherical natural color wax beads without using expensive equipment, the beads having a higher specific gravity than water so that they do not flow into the sea and being biodegradable to fundamentally remarkably reduce marine ecosystem contamination, and the spherical natural color wax beads prepared by the method.

In order to achieve the above objects, the invention provides a method for preparing spherical natural color wax beads, comprising
1) a first step of melting wax in a reaction vessel and then defoaming it;
2) a second step of adding a pigment and a filler to the reaction vessel which passes through the first step so that a mixture including the wax has a higher specific gravitythan 1.0;
3) a third step of adding water including a suspension stabilizer to the reaction vessel which passes through the second step and stirring it;
4) a fourth step of mixing the reactant which passes through the third step with a cold water to lower the temperature of the mixture below the melting temperature (Tm) of the wax, thereby forming spherical color beads; and
5) a fifth step of separating from the mixture the spherical color beads which are included in the mixture of the fourth step.

Preferably, the method for preparing spherical natural color wax beads may further optionally comprise a sixth step of cleansing the separated spherical color beads; or a seventh step of sorting them by size, subsequently to the fifth step.

Further, the invention provides spherical natural color wax beads prepared by the above method. Preferably, the specific gravity of the spherical natural color wax beads exceeds 1.0.

The method for preparing spherical natural color wax beads and the beads according to the present invention enable spherical natural color wax beads to be manufactured in large quantities without using expensive equipment, and the prepared beads have a higher specific gravity than water so that the beads do not flow into the sea and they are biodegradable to fundamentally remarkably reduce marine ecosystem contamination.

### BRIEF DESCRIPTION OF THE DRAWINGS

Fig. 1 is the figure showing a method for preparation of spherical wax beads using expensive vibrating nozzle and jet cutter equipment

### DETAILED DESCRIPTION OF THE INVENTION

The inventors, while doing researches on methods for preparing spherical natural color wax beads using a natural substance-derived wax, have found that spherical natural color beads could be prepared in large quantities, the beads having a higher specific gravity than water so that they do not flow into the sea and being biodegradable to fundamentally remarkably reduce marine ecosystem contamination, and completed the present invention.

Hereafter, the invention will be described in detail.

The method for preparing spherical natural color wax beads according to the present invention comprises
1) a first step of melting wax in a reaction vessel and then defoaming it;
2) a second step of adding a pigment and a filler to the reaction vessel which passes through the first step so that a mixture including the wax has a higher specific gravity than 1.0;
3) a third step of adding water including a suspension stabilizer to the reaction vessel which passes through the second step and stirring it;
4) a fourth step of mixing the reactant which passes through the third step with a cold water to lower the temperature of the mixture below the melting temperature (Tm) of the wax, thereby forming spherical color beads; and
5) a fifth step of separating from the mixture the spherical color beads which are included in the mixture of the fourth step.

Preferably, the method for preparing spherical natural color wax beads may further optionally comprise a sixth step of cleansing the separated spherical color beads; or a seventh step of sorting them by size, subsequently to the fifth step.

A detailed description about each step will be followed.

### 1) First Step

The first step in this invention is to melt wax in a reaction vessel and then defoam it. The wax may be a natural substance-derived wax and for example, it may include, but not be limited to, Bees wax, Berry wax, Rice Bran wax, Sunflower wax, Carnauba wax, Candelilla wax, Shellac wax, Cetyl Palmitate, Castor wax, and Vegetable wax. The wax can be melted by raising the temperature of the reaction vessel above the melting temperature (Tm) of the wax and preferably, the temperature of the reaction vessel may be maintained in the range of 60 to 99°C, preferably 70 to 95°C in the first step. Further, foams included in the wax may be defoamed by reducing the pressure of the reaction vessel and stirring them. Preferably, the defoaming process may be conducted sufficiently until no foams are generated in the reaction vessel. Also, preferably, the stirring process may be conducted sufficiently for 1 to 3 hours at 50-300 rpm.

### 2) Second Step

The second step in this invention is to add a pigment and a filler to the reaction vessel which passes through the first step so that a mixture including the wax has a specific gravity exceeding 1.0. The pigment may be any ordinary natural pigments and for example, zinc oxide may be used. Preferably, the pigment may be particles having a diameter of 0.01 to 100 µm. The pigment may be used preferably in an amount of 1 to 50 parts by weight with regard to 100 parts by weight of the wax.

The filler may be any ordinary fillers that are used for cosmetic purposes and for example, one or more selected from the group consisting of talc, mica, Ti02, and starch powder may be used, but it may not be limited thereto. Preferably, the filler may be particles having a diameter of 0.01 to 100µm. The filler may be used preferably in an amount of 1 to 70 parts by weight with regard to 100 parts by weight of the wax. Preferably, the filler may be added in such an amount that the mixture of the wax, the pigment and the filler in the second step has a specific gravity exceeding 1.0 and more preferably, the mixture has a specific gravity higher than 1.0 but not higher than 1.5. The beads prepared within the above range have a higher specific gravity than water so that the used beads do not flow into the sea and they are biodegradable to fundamentally remarkably reduce marine ecosystem contaminationand to make the cleansing and separation process of the beads easy.

Preferably, the temperature of the reaction vessel in the second step may be maintained in the range of 60 to 99°C, preferably 70 to 95°C. Further, foams included in the wax may be defoamed by reducing the pressure of the reaction vessel and stirring them. Preferably, the stirring process may be conducted sufficiently for 1 to 3 hours at 50-300 rpm.

Further, in the second step, the pigment and the filler may be added simultaneously and defoamed, or any one of the pigment and filler may be added first and defoamed and then the other may be added.

### 3) Third Step

The third step in this invention is to add water including a suspension stabilizer to the reaction vessel which passes through the second step and stir it. The suspension stabilizer may be any known suspension stabilizers and for example, poly vinyl alcohol may be used. The temperature of the water including the suspension stabilizer to be added to the reaction vessel in the third step may be maintained in the range of 60 to 99°C, preferably 70 to 95°C and more preferably, it may be the same as or higher than the temperature of the wax that passes through the second step. The wax beads prepared in this way have excellent sphericity. The amounts of the suspension stabilizer and the water to be added in the third step may be preferably 10 to 100 weights by part of the suspension stabilizer and 300 to 600 parts by weight of the water with regard to 100 parts by weight of the wax. The wax beads prepared in this way have excellent sphericity and color stability. The stirring in the third step may be conducted preferably at a speed of 50 to 500 rpm for 30 min. to 3 hours. If the stirring speed is too slow, the sphericity of the prepared wax beads may be deteriorated, and if the stirring speed is too fast, the size of the particles may become too smaller.

### 4) Fourth Step

The fourth step in this invention is to mix the reactant which passes through the third step with a cold water to lower the temperature of the mixture below the melting temperature (Tm) of the wax, thereby forming spherical color beads. The temperature and the amount of the cold water may be adjusted in such a degreethat the temperature of the mixture in the fifth stepbecomes lower than the melting temperature of the wax and preferably, the temperature of the cold water may be 1 to 10°C so that the temperature of the mixture can shortly become below 50°C. In this case, the amount of the water to be used can be reduced and the sphericity of the wax beads to be prepared is excellent.

### 5) Fifth Step

The fifth step in this invention is to separate from the mixture the spherical color beads which are included in the mixture of the fourth step. Any ordinary separation methods can be applied. Particularly, the preparation method of the invention can make the separation process easier because the specific gravity of the prepared beads is higher than water.

The method for preparing spherical natural color wax beads of the invention may further optionally comprise a sixth step of cleansing the separated spherical color beads; or a seventh step of sorting them by size, respectively or simultaneously, subsequently to the fifth step. Through the cleansing process of the sixth step, the suspension stabilizer which might remain in the wax beads can be rinsed off, and the prepared color beads can be categorized by sorted size through the seventh step.

A preferred preparation method of the invention may be as follows.

60 Parts by weight of natural wax is added to a first reaction vessel equipped with a heating device, a cooling device and a stirring device, the temperature of the reaction vessel is raised to 80°C to melt the wax and then, under a reduced pressure, the wax is stirred for 3 hours at 150 rpm to defoamuntil no foams are generated. After that, the reduced pressure is removed, 5 parts by weight of zinc oxide as a pigment and 35 parts by weight of talc or mica as a filler are added so that the specific gravity of the mixture consisting of the wax, pigment and filler exceeds 1 and then, it is stirred for 2 hours at 150 rpm at 80°C under a reduced pressure to defoam until no foams are generated. 400 Parts by weight of water and 15 parts by weight of the suspension stabilizer are added to a second reaction vessel different from the first reaction vessel and after the temperature is raised to 85°C, they are stirred at 150 rpm for melting. The water including the suspension stabilizer of the second reaction vessel is added to the first reaction vessel, which is then stirred at a stirring speed of 150 rpm for 2 hours. Thereafter, 500 to 800 parts by weight of the cold water of 1 to 10°C is added so that the temperature of the reaction vessel becomes 50°C, thereby to form color beads. Thereafter, the prepared color beads are separated from the reaction vessel and optionally, they go through cleansing and sorting process to finally produce color beads having a specific gravity higher than 1 and the size of 1 to 5000µm.

Further, the present invention provides natural color wax beads prepared through the above method, wherein the natural color wax beads have such a structure that the natural pigment and the filler exist as a core structure in the center of beads and the natural wax-derived wax exists outside the core as a shell structure so that they have excellent sphericity and superior color stability; as they consist of natural substances only, they have excellent stability to humans; and they have a higher specific gravity than water so that they do not flow into the sea and they are biodegradable to fundamentally remarkably reduce marine ecosystem contamination. The particle size of the natural color wax beads of the invention may be optionally adjusted and preferably, it may be 1 to 5000µm.

The natural color wax beads of the invention may be used to replace in whole or in part the products in which previous plastic beads or wax beads have been used and for example, they may include children's products such as children's shampoos, children's lotions and creams, and children's oils; bath oils, bath pills, bath salts, bath capsules, bubble bath, body cleanser, and other bath products; eye brows, eye liners, eye shadows, mascaras, eye makeup removers, and other eye makeup products; perfumes, powder perfumes, perfumed sachets, colognes, other fragrant products; hair tints, hair color sprays, and other hair-dye products; blushers, face powders (cakes), foundations (liquids, creams, cakes), makeup bases, makeup fixatives, lipsticks (creams, pencils), lip glosses, and other makeup products (including body painting and make-up products); hair conditioners, hair tonics, hair grooming aids, hair creams, hair oils, pomades, hair sprays, hair mousses, shampoos, rinses, permanent waves, hair straighteners, and other hair products; base coats and under coats, nail polishes, nail enamels, top coats, nail creams (liquids, lotions), nail polish removers, nail enamel removers, and other manicure products; aftershave lotions, men's talcum, pre-shave lotions, shaving creams, beard softeners, and other shaving products; and soft skin lotions, massage creams, skin nutrition lotions, astringents lotions, nutrition oils (liquids), powders, cleansing cosmetics, body cosmetics (lotions, oils, creams, powders), packs, eye creams, and other base makeup products, but not be limited thereto. Preferably, the natural color wax beads of the invention may be used as scrubbing beads in products causing scrubbing effects and their content may be suitably adjusted to the purpose of products within the range of 0.1 to 20 % by weight of the final products.

## Claims

1. A method for preparing spherical natural color wax beads, comprising
1) a first step of melting wax in a reaction vessel and then defoaming it;
2) a second step of adding a pigment and a filler to the reaction vessel which passes through the first step so that a mixture including the wax has a higher specific gravity than 1.0;
3) a third step of adding water including a suspension stabilizer to the reaction vessel which passes through the second step and stirring it;
4) a fourth step of mixing the reactant which passes through the third step with a cold water to lower the temperature of the mixture below the melting temperature (Tm) of the wax, thereby forming spherical color beads; and
5) a fifth step of separating from the mixture the spherical color beads which are included in the mixture of the fourth step.

2. The method for preparing spherical natural color wax beads of claim 1, wherein the temperature of the reaction vessel in the first, second and third steps is 70 to 95°C.

3. The method for preparing spherical natural color wax beads of claim 1, wherein the pigment is 1 to 50 parts by weight with regard to 100 parts by weight of the wax.

4. The method for preparing spherical natural color wax beads of claim 1, wherein the filler is one or more selected from the group consisting of talc, mica, and starch powder.

5. The method for preparing spherical natural color wax beads of claim 1, wherein the filler is 1 to 70 parts by weight with regard to 100 parts by weight of the wax.

6. The method for preparing spherical natural color wax beads of claim 1, wherein the filler is added in such an amount that the mixture consisting of the wax, the pigment and the filler has a specific gravityhigher than 1.0 but not higher than 1.5.

7. The method for preparing spherical natural color wax beads of claim 1, wherein the temperature of the water including the suspension stabilizer to be added to the reaction vessel in the third step is the same as or higher than the temperature of the wax which passes through the second step.

8. The method for preparing spherical natural color wax beads of claim 1, wherein the temperature of the cold water in the fourth step is 1 to 10°C.

9. Spherical natural color wax beads prepared by the method described in any one of claims 1 to 8.

10. The spherical natural color wax beads of claim 9, wherein the color wax beads have a specific gravityhigher than 1.0 but not higher than 1.5.

11. A scrubbing product comprising the spherical natural color wax beads described in claim 9.
